# EUROPEAN PATENT APPLICATION

(11) **EP 4 834 203 A1**
(43) Date of publication of application: **04.11.2026**
(21) Application number: 25173506.4
(22) Date of filing: 30.04.2025
(51) Int. Cl.: A61B 5/00, G16H 30/00

(54) **CLASSIFYING WHOLE SLIDE IMAGES TOWARDS IMAGE ANALYSIS ALGORTHIMS IN PATHOLOGY**

(71) Applicant: Koninklijke Philips N.V., 5656 AE Eindhoven (NL)
(72) Inventor: KRISHNAIYER RAMAN, Sivaramakrishnan, Eindhoven (NL); KUMAR, Prashant, Eindhoven (NL); PALIWAL, Shreya, Eindhoven (NL)
(74) Representative: Philips Intellectual Property & Standards

(57) **Abstract**

A computing device (46) having access to a plurality of sources. The computing device includes a memory (52) storing instructions (24) and a processor (48) configured to execute the instructions to cause the processor to access one or a combination of the plurality of sources for metadata associated with plural images of digitally-scanned slides (74), filter the metadata, using user-configured classification rules corresponding to artificial intelligence(AI) algorithms, to direct processing of at least one of the plural images to one of the AI algorithms (76), and schedule execution of the one of the AI algorithms to process the at least one of the plural images without a need for user intervention (78).

## Description

### FIELD OF THE INVENTION

The present invention is generally related to pathology, and more particularly, to artificial intelligence-assisted digital pathology.

### BACKGROUND OF THE INVENTION

In digital pathology, digital images of biopsy tissue slices are used for diagnosis by a pathologist. A system, such as the Philips Image Management System (IMS), manages these images, which can be viewed by pathology personnel with an image viewer.

Increasingly, artificial intelligence (AI) algorithms are aiding pathologists in developing diagnoses. Various AI algorithms exist, but not all algorithms are applicable in a particular context because each AI algorithm may be specific to tissue type, stain type, specimen type, and sub-tissue type, etc.

The workflow for integrating AI into the digital pathology workflow is tedious and inefficient. For instance, existing systems may only enable manually selecting algorithms to be executed on images, and then the pathologist needs to wait for the results. Additionally, the metadata used by an algorithm may not be available from a system that manages patient data and related laboratory processes (e.g., a Lab Information System (LIS)).

### SUMMARY OF THE INVENTION

It would be advantageous to improve the scheduling of execution of different artificial intelligence (AI) algorithms as applied to images of digitally scanned slides. To better address one or more of these concerns, in a first aspect of the invention, a computing device is presented that has access to a plurality of sources. The computing device includes a memory storing instructions and a processor configured to execute the instructions to cause the processor to: access one or a combination of the plurality of sources for metadata associated with plural images of digitally-scanned slides, filter the metadata, using user-configured classification rules corresponding to artificial intelligence (AI) algorithms, to direct processing of at least one of the plural images to one of the AI algorithms, and schedule execution of the one of the AI algorithms to process the at least one of the plural images without a need for user intervention. For instance, the selection and scheduling of AI algorithms is a manually-intensive process. A pathologist needs to consider a plurality of different parameters, including procedure type, stain type, tissue type, among others, and have knowledge of AI algorithms that are suitable for a given set of parameter inputs. Such tasks are further complicated by the extensive, daily case load (e.g., approximately 150 or more cases) of the pathologist. For a given set of digitally-scanned slides, certain embodiments filter the information, and match the inputs to route the workflow to a suitable AI algorithm(s) based on the configured classification rules without the need for user intervention. By doing so, the pathology workflow is streamlined, and the pathologist is significantly less burdened administratively, enabling more time for substantive tasks (e.g., analysis of the AI results) while reducing the risk of error in performing these substantive tasks.

In one embodiment, the processor is further configured to execute the instructions to: determine that one of the plurality of sources is incapable of fully providing the metadata needed for execution of the one of the AI algorithms; and responsive to the determination, cause the processor to access the combination of the plurality of sources. For instance, in some cases, even if a source (e.g., Lab Information System) is operably integrated into a system for analyzing digitally-scanned slides, not all of the information necessary for implementing a given AI algorithm may be available. Such an omission in information may cause further delay and/or user intervention, whereas certain embodiments enable access to additional sources needed to execute a given AI algorithm without delay or user intervention.

In one embodiment, the processor is further configured to execute the instructions to provide a (GUI) arranged to enable a user to configure the classification rules, the GUI comprising: a first menu providing a list of the AI algorithms; and additional menus corresponding to parameters needed to execute the respective AI algorithms, each of the additional menus corresponding to the parameters and comprising a plurality of options. The GUI is arranged to enable a simple and effective, one-time (or on demand) configuration of the classification rules for the AI algorithms.

In one embodiment, the processor is further configured to execute the instructions to auto-populate the additional menus based on user selection of one of the AI algorithms from the list, the auto-population comprising a selection from among the plurality of options and subsequent listing of the selected options among the additional menus that are needed to execute the one of the AI algorithms selected from the list. The auto-population mechanisms of certain embodiments facilitates the configuration of the classification rules.

These and other aspects of the invention will be apparent from and elucidated with reference to the embodiment(s) described hereinafter.

### BRIEF DESCRIPTION OF THE DRAWINGS

Many aspects of the invention can be better understood with reference to the following drawings, which are diagrammatic. The components in the drawings are not necessarily to scale, emphasis instead being placed upon clearly illustrating the principles of the present invention. Moreover, in the drawings, like reference numerals designate corresponding parts throughout the several views.
FIG. 1 is a schematic diagram of an example rule engine configuration interface for selecting specific input attributes, in accordance with an embodiment of the invention.
FIG. 2 is a schematic diagram that illustrates an example user configuration of a classification rule with valid and applicable attributes for the algorithm selected in the interface of FIG. 1, in accordance with an embodiment of the invention.
FIG. 3 is a schematic diagram that illustrates an example screen showing user-configured classification rules for several algorithm types based on different attributes, in accordance with an embodiment of the invention.
FIG. 4 is a schematic diagram that illustrates example rule engine based slide image filtering with AI algorithm integration, in accordance with an embodiment of the invention.
FIG. 5 is a schematic diagram that illustrates an example slide image filtering and classification workflow based on user input to the rule engine configuration interface of FIG. 1, in accordance with an embodiment of the invention.
FIG. 6 is a block diagram that illustrates an example computing device for implementing rule engine configurations and filtering and scheduling execution of AI algorithms on the basis of the rule engine configurations, in accordance with an embodiment of the invention.
FIG. 7 is a flow diagram of an example method of providing a user interface for rule engine configuration for AI algorithms, in accordance with an embodiment of the invention.
FIG. 8 is a flow diagram of an example method for scheduling execution of AI algorithms, in accordance with an embodiment of the invention.

### DETAILED DESCRIPTION OF EMBODIMENTS

Disclosed herein are certain embodiments of a rule engine configuration interface and associated rules-based filtering and scheduling methods that provide a tool for creating workflow rules related to filtering (e.g., routing or directing) and scheduling the processing by, or execution of, suitable artificial intelligence (AI) algorithms for application to corresponding slide images. In one embodiment, a user may use a graphical user interface (GUI) to create workflow classification rules, for each AI algorithm, that define what attributes (e.g., sample type, tissue type, sub-tissue type, stain type, etc.) are needed for the particular AI algorithm to be able to analyze images of digitally-scanned slides. The use of the rule engine configuration interface serves to address inefficiencies resulting from purely manual AI algorithm selection solutions in existing digital pathology tools, and also help to reduce the risk of error by offloading a significant portion of the digital pathology workflow to enable more time for a pathologist or other personnel to make a determination about the images (e.g., diagnosis, prognosis, etc.).

Digressing briefly, with an increasing adoption of AI algorithms in the digital pathology workflow, pathology groups (e.g., pathology personnel, including pathologists, technicians, pathology assistants, etc.) have shown increased interest in having the ability to integrate multiple AI algorithms in their clinical workflow. Generally, AI algorithms work in a specific context that is dependent on certain attributes. Attributes, as further explained below, include tissue type, stain type, age group, specimen type, sub-tissue type, etc. Since a given AI algorithm is primarily applicable to specific cases, or even specific slides within a case, a pathologist has to determine the applicability of the algorithm to the case, and manually trigger algorithm execution. Such existing workflow processes are not only tedious, but may also limit the efficiency of the digital pathology workflow (e.g., delay review by a pathologist). Further, improvement upon existing methods involving AI algorithms in digital pathology workflows does not pose a trivial task, since it is often the case that metadata needed for the AI algorithm is not available from existing patient/lab management systems (e.g., Lab Information System (LIS)). Also, the permutation combinations of contexts for the applicable algorithms vary across the specialty, or sub-specialty, of pathology cases, furthering the challenge of manual configurations, particularly given the large workload a pathologist is presented with each day (e.g., a hundred or more cases). Burdening the pathologist with the selection and scheduling of AI algorithms for analysis of slide images may also leave less time for analysis by the pathologist, which may lead to errors. In contrast, certain embodiments of a rule engine configuration interface and associated filtering/scheduling methods address these and/or other problems. For instance, through filtering mechanisms, digital slides images are routed to a suitable AI algorithm and then scheduled for execution without any user intervention needed, enabling AI results to be made available as soon as, for instance, the pathologist opens the case. Such efficiency in preprocessing of the incoming digital slide images reduces user involvement, and enables more time for the pathologist to focus on substantive matters such as diagnosis, prognosis, etc.

Having summarized certain features of a rule engine configuration interface and associated methods of the present disclosure, reference will now be made in detail to the description of a rule engine configuration interface and associated methods as illustrated in the drawings. While a rule engine configuration interface and associated methods will be described in connection with these drawings, there is no intent to limit it to the embodiment or embodiments disclosed herein. For instance, there is no intent to limit the AI algorithms to a certain type, understanding that AI algorithms may take the form of any of a plurality of AI algorithms available to the industry. AI algorithms include fully convolutional neural networks (FCNN), convolutional neural networks, support vector machines, decision trees, random forests, K-nearest neighbor, foundation models (e.g., trained on a very large set of unlabeled medical data that then can be fine-tuned with a minimal amount of labeled data, etc., similar to Virchow), among others. Also, though emphasis is placed on digital pathology solutions that include AI orchestration, other use scenarios are contemplated, including incorporation of the disclosed embodiments into existing image management system, and/or integration with different third-party AI solutions that enables diagnostic (histopathology, molecular, etc.) and prognostic use cases related to pathology to get relevant information on the patient, slide and clinical context.

Further, although the description identifies or describes specifics of one or more embodiments, such specifics are not necessarily part of every embodiment, nor are all of any various stated advantages necessarily associated with a single embodiment. On the contrary, the intent is to cover alternatives, modifications and equivalents included within the principles and scope of the disclosure as defined by the appended claims. For instance, two or more embodiments may be interchanged or combined in any combination. Further, it should be appreciated in the context of the present disclosure that the claims are not necessarily limited to the particular embodiments set out in the description.

Before commencing a detailed description of the drawings, reference to attributes, or likewise, parameters, needed or used by the AI applications for processing pathology slides includes sample type, tissue type, sub-tissue type, stain type, and clinical context, among others as would be understood by one having ordinary skill in the art. For instance, slide label information typically includes the type of sample being analyzed, such as biopsy, resection, or cytology specimen. This attribute helps the algorithm understand the nature of the tissue and the expected structures. The tissue type refers to the specific type of tissue present on the slide, such as breast tissue, lung tissue, or skin tissue. Different tissues have unique histological features, and the algorithm should be trained to recognize these features accurately. In some cases, the slide label may specify a sub-tissue type within a larger tissue category. For example, within breast tissue, the sub-tissue type may be ductal or lobular structures. Such level of detail helps the algorithm focus on specific features relevant to the analysis. In cases where specific stains are used to highlight certain features or structures, the slide label may include information about the stain type. This attribute may facilitate interpretation of the colors and patterns in the image by the algorithm. Slide label information may also include clinical context, such as the suspected diagnosis, relevant symptoms, or previous treatment history. This attribute may guide analysis by the algorithm and help the algorithm to generate more relevant insights.

The following description will describe several stages in the implementation and execution of certain embodiments of a rule engine configuration interface and associated methods, including establishing AI algorithm filtering criteria (stage 1), preprocessing slide information (stage 2), and extracting and provisioning configured features towards the AI algorithm (stage 3) based on one or more attributes. Stage 1 involves the configuration of classification rules corresponding to AI algorithms (e.g., sourced by the imaging management entity, hospital or research facilities, and/or third party vendors) and expected parameters (attributes) by the algorithm for successful processing of the slides for AI analysis.

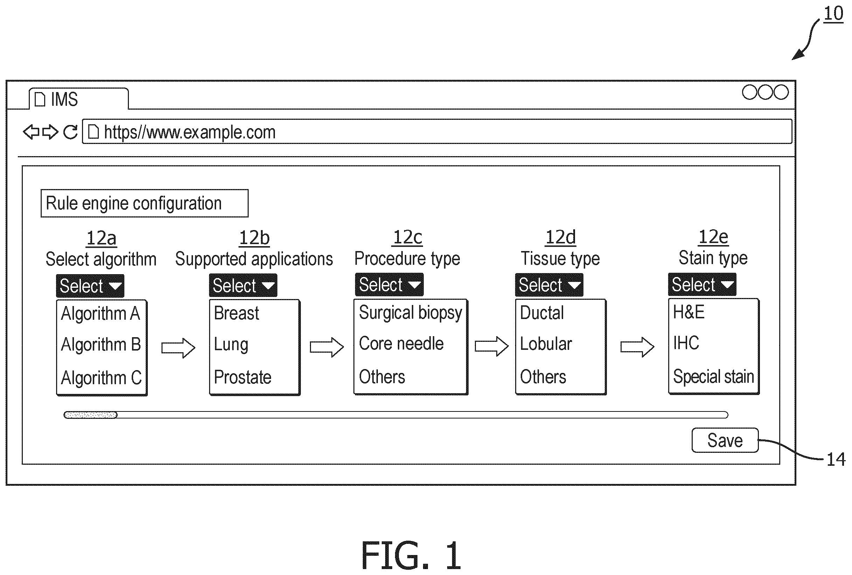

Referring to FIG. 1, shown is an embodiment of an example rule engine configuration interface 10 that may be presented on a display device integral to, or otherwise coupled to (e.g., wired or wireless), a computing device. The rule engine configuration interface 10 enables selection of algorithms (e.g., AI algorithms) and auto-population of expected parameters (e.g., attributes) resulting from the selection of a given algorithm. Parameters/attributes may include tissue type, procedure type, stain type, among others as explained herein. Though there are four parameters/attributes shown in association with selection of the algorithms, it should be appreciated by one having ordinary skill in the art that the list of parameters/attributes may be extended to any additional information within a patient, image and/or clinical context based on the nature of the selected menus.

In one embodiment, the rule engine configuration interface 10 includes a plurality of menus 12 that are configured as drop-down menus. Note that in some embodiments, other user interface formats for providing a user a choice of AI algorithms and the resultant auto-population of menus corresponding to parameters/attributes may be presented in an interface. Further, in some embodiments, an option may be presented (e.g., a custom button icon, not shown) that enables a user to configure a custom classification rule that includes information beyond that available from the menus 12, or in some embodiments, that may include at least some attributes from the menus 12. In some embodiments, the rule engine configuration interface 10 enables the user to edit the auto-populated selections. Though a single interface with plural menus 12 are shown, in some embodiments, each interface may have its own dedicated menu 12 with arrow icons or pagination mechanisms that enable a user to proceed to a next interface (e.g., next page) for selecting from the next menu 12 (or which automatically advance to the next interface after selection of a given menu option).

In the depicted embodiment, the menus 12 include an algorithm menu 12a, as well as additional menus 12b-12e corresponding to parameters/attributes that are needed by, or used by, the algorithms. The additional menus 12b-12e include supported applications menu 12b, procedure type menu 12c, tissue type menu 12d, and stain type menu 12e. The algorithm menu 12a includes a plurality of algorithm options (e.g., Algorithm A, Algorithm B, Algorithm C, etc.). The supported applications menu 12b includes a plurality of applications (options) the algorithms in the algorithm menu 12a support (e.g., breast, lung, prostate, etc.). The procedure type menu 12c includes plural procedure options that are supported by the algorithms in the algorithm menu 12a, and in this example, include surgical biopsy, core needle, and others (i.e., others as a selectable, user-specified option, or others meaning other types may be pre-listed). In some embodiments, selection of the others option enables the manual entry by a user of a particular option. The tissue type menu 12d includes plural options for tissue type that are expected for the algorithms of the algorithm menu 12a, and include ductal, labular, and/or others. The stain type menu 12e includes plural stain type options that may be used for the algorithms of the algorithm menu 12a, including H&E, IHC, and others. The rule engine configuration interface 10 further includes a save icon 14 for selection by the user subsequent to selection of the various menu options.

Note that selection (e.g., of an option in the algorithm menu 12a) herein may refer to manual selection, such as touch-screen type selection or cursor-type selection, and/or in some embodiments, automatic selection by the underlying software. The rule engine configuration interface 10 may include additional and/or other features that facilitate navigation to the selectable options. In one embodiment, the rule engine configuration interface 10 is intended as a one-time, initial configuration of classification rules that are later used to route incoming slide images to the appropriate AI algorithm, though it should be appreciated that edits to the classification rules may be made later (e.g., adding, deleting, reconfiguring, etc.).

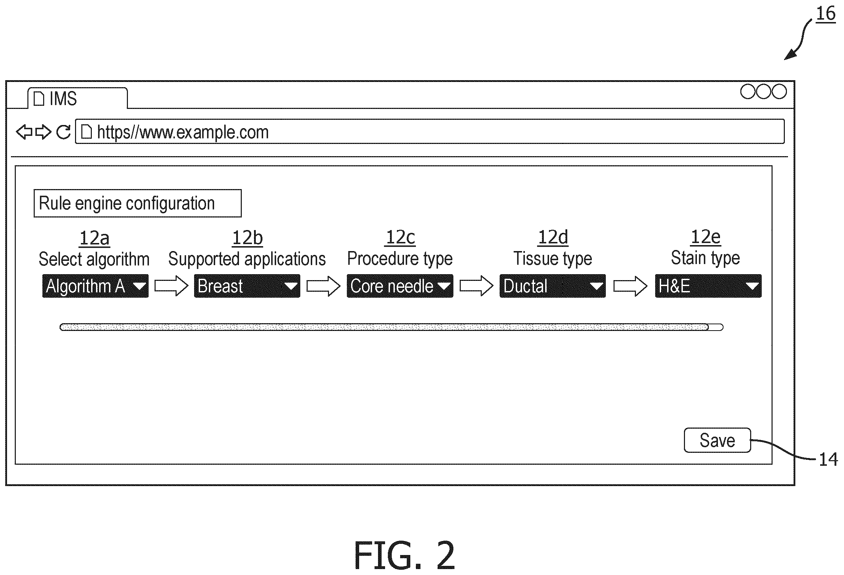

In one embodiment, the selection of one of the algorithms from the algorithm menu 12a triggers an auto-population of the rest of the menus 12b-12e. For instance, in stage 1, parameters are automatically selected for creating classification rules for a user-selected algorithm. Referring to FIG. 2, shown is an embodiment of an example rule engine configuration 16 (e.g., an interface) that self or auto-populates menus 12b-12e responsive to user selection of an algorithm in menu 12a (e.g., Algorithm A). The underlying configuration process has knowledge of the AI algorithms and their requisite parameters for execution, and automatically populates the menus 12b-12e based on the option selected in the algorithms menu 12a. In this example, the selection of Algorithm A in menu 12a prompts auto-population/auto-selection of the breast option for the supported application menu 12b, the core needle option for the procedure type menu 12c, the ductal option for the tissue type menu 12d, and the H&E option for the stain type menu 12e. At this point, the user can save the rule engine configuration for Algorithm A via selecting the save icon 14 for later use in filtering/scheduling, and/or return for rule engine configuration for additional algorithms.

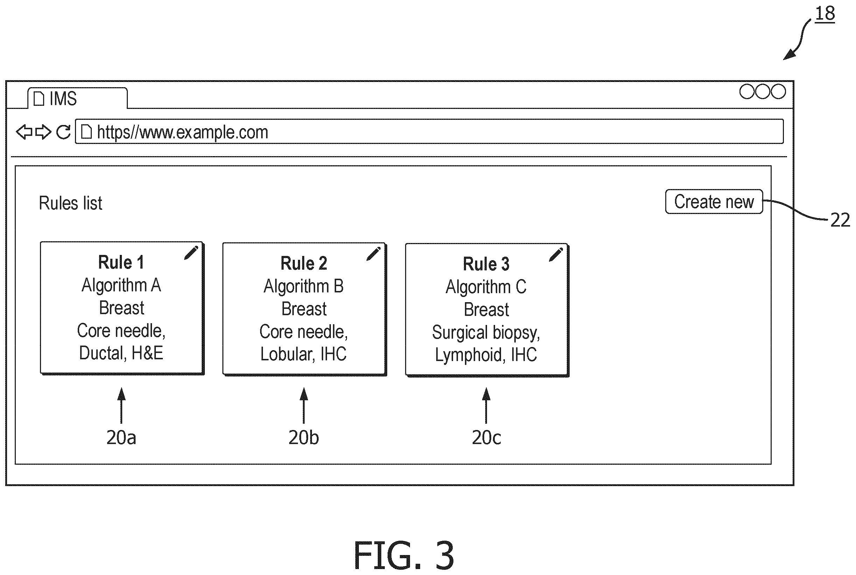

As a result of the configurations of stage 1, plural rules may be created for each algorithm type with the applicable parameters for each algorithm, as shown in FIG. 3. FIG. 3 shows an embodiment of an example rules list 18 (an interface) that lists plural configured classification rules 20 (e.g., 20a, 20b, 20c) resulting from the stage 1 processing described above. In this example, the rules list 18 includes classification rule 1 20a, classification rule 2 20b, and classification rule 3 20c. Each classification rule corresponds to one of the AI algorithms selected from the algorithms menu 12a and the corresponding parameters used by the selected AI algorithm (e.g., based on the auto-populated menus 12b-12e). Each of the classification rules 1, 2, and 3 include the parameters used by, or needed by, the corresponding AI algorithm to process a given slide image. Classification rule 1 20a includes algorithm A, with attributes for supported applications, procedure type, tissue type, and stain type including breast, core needle, ductal, and H&E, respectively. Classification rule 2 20b includes algorithm B and corresponding attributes of breast, core needle, labular, and IHC. Classification rule 3 20c includes algorithm C, and corresponding attributes of breast, surgical biopsy, lymphoid, and IHC. In some embodiments, the rules list 18 may include additional options, including a create new button icon 22 for creating new rules.

As explained above, a next stage (stage 2) includes pre-processing slide information to identify the source(s) of information. The expected slide information used by an AI algorithm, including sample type, tissue type, etc., should be gathered from one or, if needed, a combination of sources to ensure all the pre-requisite parameter inputs are available for processing the slide towards the next stages. One source of slide information may include a patient/laboratory process management system, such as Laboratory Information Systems (LIS). Another source may include reports from procedures performed for extracting the tissue sample, such as biopsy procedure reports and/or grossing or macroscopic examination reports. In the absence of such information from these aforementioned sources, the system may process the incoming slide to extract morphological features of cells and tissues in the image using their shape, structure and appearance to identify slide information (e.g., stored in a storage device, such as a memory).

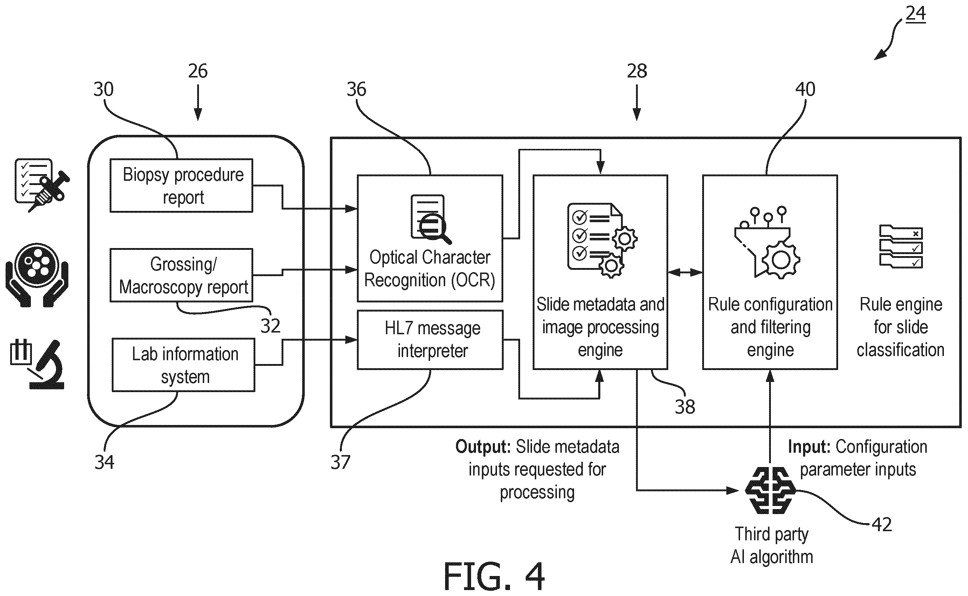

FIG. 4 schematically illustrates an embodiment of an example rule engine slide preprocessing and filtering process with AI integration 24. The rule engine slide preprocessing and filtering process with AI integration 24 includes sources 26 and the rule engine 28 for filtering slide images using the classification rules (e.g., configured as described in association with FIGS. 1-2), which enables the rule engine slide preprocessing and filtering process with AI integration 24 to route the slide images to the appropriate (suitable) AI algorithm. The sources 26 include a biopsy procedure report 30, grossing/macroscopy report 32, and lab information system (LIS) 34. The biopsy procedure report 30 includes a detailed document prepared by a pathologist after examining a sample of tissue that has been removed from a patient during a biopsy procedure. One purpose of the biopsy procedure report 30 is to provide a definitive diagnosis of the tissue sample, which may help guide further medical treatment and/or management. In the rule engine 28, text mining or Optical Character Recognition (OCR) 36 may be used for reading the digitized physical report and interpretation of needed parameters.

A grossing report, also known as a macroscopic examination report, and referred to herein as the grossing/macroscopy report 32, includes a detailed description of the tissue specimen as observed with the naked eye, before microscopic examination. This report 32 is typically prepared by a pathologist or a trained technician, and is an important part of the biopsy evaluation process. In the rule engine 28, text mining or OCR 36 may be used for reading the digitized physical report and interpretation of needed parameters, similar to that discussed above for the reading/interpretation of the biopsy procedure report 30.

The laboratory information system (LIS) 34 includes computer software that processes, stores and manages data from patient medical processes and tests. In the rule engine 28, standard HL7 based communication protocol 37 may used for information exchange.

Though not shown in FIG. 4, in some embodiments, an additional source may be storage (e.g., local or remote) of metadata that is based on image analysis of the slide image (e.g., where metadata from the other sources is unavailable).

Note that there exists a hierarchy of pre-processing of slide information for AI algorithms. In one embodiment, the hierarchy is preset or preconfigured, though in some embodiments, may be user-configurable. In one embodiment, the hierarchy is established based on the ease or difficulty in obtaining the information, which priority going from easy to difficult. In one embodiment, the hierarchy involves the following: (a) available LIS parameter in standard interoperable format; (b) available LIS parameter but in non-standard format (e.g., method for configurable mapping of non-standard formats/data to expected input); (c) not available as a LIS parameter but usually available in associated reports as free text (e.g., method for text mining/optical character recognition to extract expected input); and (d) not available as a LIS parameter but interpreted based on image content (e.g., method for image processing to extract expected input).

A next stage (stage 3) in the processing of slide images includes extracting and provisioning configured features towards the AI algorithm. This stage enables the extraction of information needed for the AI algorithms to execute without user intervention, including the patient/case information (e.g., metadata) and the corresponding slide image associated with the patient case. Continuing with the example illustration of FIG. 4, the pre-processed source information, or metadata, is fed to the slide metadata and image processing engine 38. The slide metadata and image processing engine 38 exchanges this information with the rule configuration and filtering engine 40 and outputs slide metadata inputs for processing by the AI algorithm 42. The AI algorithm 42, though depicted in FIG. 4 as a 3^{rd} party AI algorithm, may be sourced from any entity (e.g., 3^{rd} party or otherwise), and is included as part of the classification rules configured by a user as explained in association with FIGS. 1-3. The rule configuration and filtering engine 40 includes the classification rules and is used, in conjunction with the slide metadata and image processing engine 38, to filter the slide images. The slide metadata and image processing engine 38, based on the saved classification rules, filters (e.g., directs or routes) the slide image and slide image metadata to a suitable AI algorithm 42, and schedules the execution of the AI algorithm (e.g., for processing of the slide image). In one embodiment, the slide metadata and image processing engine 38 determines if there is an appropriate match (e.g., exact match in parameters of the classification rules with the single or multi-sourced metadata) for determining a suitable AI algorithm that applies to the received metadata. Based on the filtering (e.g., based on a match), execution of the AI algorithm is scheduled (e.g., immediately, which may occur before the pathologist opens the case, or in some embodiments, based on a triggering condition or event, such as detecting access to a given case file, time lapse, etc.). The slide metadata and image processing engine 38 further includes image processing functionality for extracting morphological features from an image and storage thereof. For instance, any of a plurality of available image processing techniques may be used to extract morphological features like cellular, tissue architecture for recognizing patterns across different incoming slide images using their shape, structure and appearance. Different slide images are identified using an image processing technique(s), and needed slide information is extracted. Note that functionality of the slide metadata and image processing engine 38 and rule configuration and filtering engine 40 may be combined in some embodiments, or functions may be shared amongst both in some embodiments.

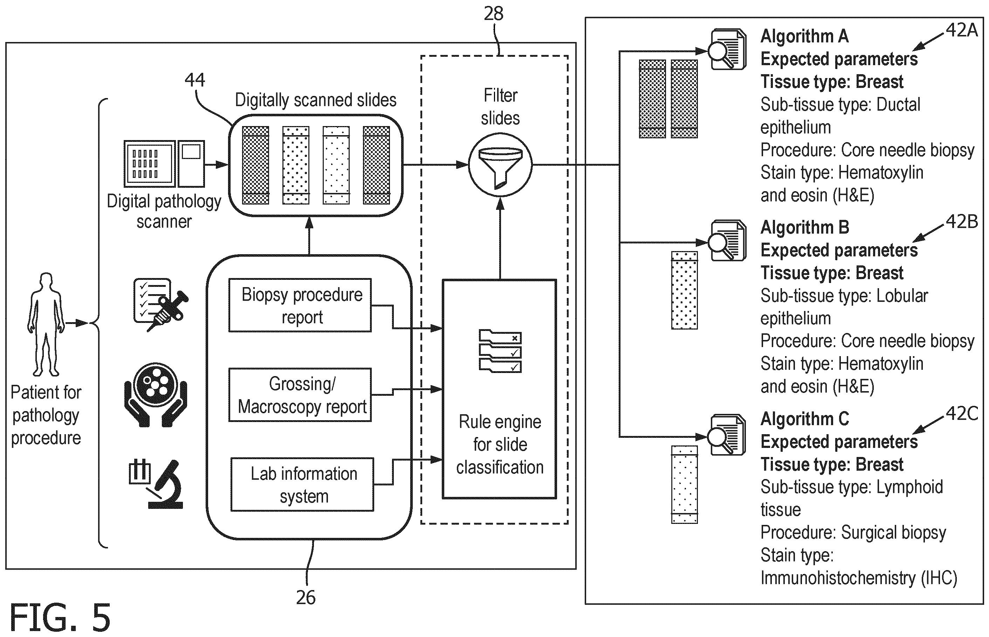

Referring to FIG. 5, shown is an example of stages 1-3 in operation. Based on the hierarchy and information available in each source 26, the extraction process is initiated (e.g., using OCR 36 and/or HL7 37), followed by stage 2 and stage 3 processing. FIG. 5 includes some components from FIG. 4, including the sources 26, the rule engine 28, and additionally, digitally scanned slide images 44 along with different algorithms 42. The different algorithms 42 include algorithms 42A, 42B, and 42C corresponding respectively to algorithm A, algorithm B, and algorithm C associated with the different classification rules (e.g., FIG. 3).

In one example use case, and still referring to FIG. 5, a typical breast surgical biopsy may involve excision of the primary tumor from a breast and removal of the axillary lymph nodes. When the sample gets processed in a pathology lab, the processing results in multiple slide images 44 corresponding to multiple slides, with a few coming from the breast tissue and few from the lymph nodes. Post-staining and scanning, all the slide images may be available under the same patient case in the LIS (and an imaging management system (IMS)). If the pathologist wants hormone receptor (ER/PR) AI evaluation done, then only the breast tissue slides are eligible for analysis. Lymph node slides are not eligible. To have an automated workflow towards AI analysis of incoming slides, a mechanism to distinguish breast tissue slides from lymph node slides is needed. Similar to that described above in association with FIG. 4, the sources are pre-processed (e.g., OCR and/or interpretation) and the rule engine 28 (e.g., using slide metadata and image processing engine 38 and rule configuration and filtering engine 40) filters the slide images using the pre-processed metadata and the classification rules and routes the incoming slide images to the appropriate AI algorithms. As shown in FIG. 5, some of the slide images are evaluated using Algorithm A 42A, one of the slides using algorithm B 42B, and one of the slides using algorithm C 42C. Algorithm A 42A includes expected parameters of breast tissue type, ductal epithelium sub-tissue type, using core needle biopsy and a stain type of H&E. Algorithm B 42B includes expected parameters of breast tissue type, labular epithelium sub-tissue type, using core needle biopsy and a stain type of H&E. Algorithm C 42C includes expected parameters of breast tissue type, lymphoid tissue as the sub-tissue type, using surgical biopsy and a stain type of IHC.

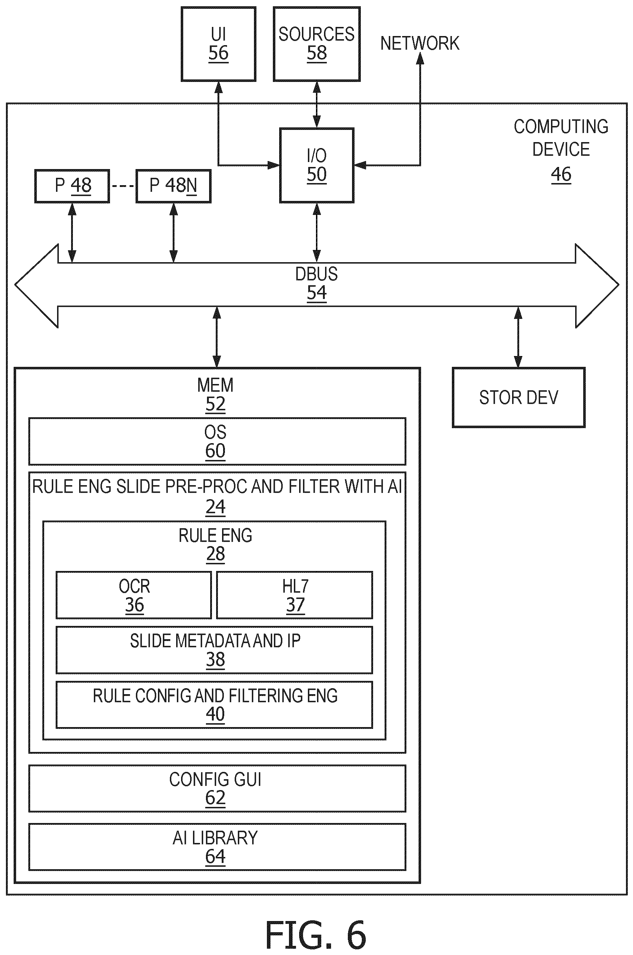

FIG. 6 is a block diagram of an embodiment of example computing device 46, which may be used to implement embodiments of rule engine configuration interface and associated methods. In the depicted embodiment, the computing device 46 is illustrated as a computer or computing system, though in some embodiments, the computing device 46 may be a sub-component(s) of a computing system, or functionality of the same may be distributed among multiple devices or computing systems. The computing device 46 includes one or more processors 48 (e.g., 48A...48N), input/output interface(s) 50, and memory 52, etc. coupled to one or more data busses, such as data bus 54. The processor(s) 48 may be embodied as a custom-made or commercially available processor, including a single or multi-core central processing unit (CPU), tensor processing unit (TPU), graphics processing unit (GPU), vector processing unit (VPU), or an auxiliary processor among several processors, a semiconductor-based microprocessor (in the form of a microchip), a macroprocessor, one or more application specific integrated circuits (ASICs), field programmable gate arrays (FPGAs), a plurality of suitably configured digital logic gates, and/or other existing electrical configurations comprising discrete elements both individually and in various combinations to coordinate the overall operation of the computing device 46.

The I/O interfaces 50 comprise hardware and/or software to provide one or more interfaces to various sub-systems, including to one or more user interface(s) 56 (which may include user input devices and one or more display devices) and sources 58 (e.g., sources 26), including image/slide sources 44, biopsy procedure report 30, gross/macroscopy report 32, and LIS 34. The I/O interfaces 50 may also include additional functionality, including a communications interface for network-based communications. For instance, the I/O interfaces 50 may include a cable and/or cellular modem, and/or establish communications with other devices or systems via an Ethernet connection, hybrid/fiber coaxial (HFC), copper cabling (e.g., digital subscriber line (DSL), asymmetric DSL, etc.), using one or more of various communication protocols (e.g., TCP/IP, UDP, etc.). In general, the I/O interfaces 50, in cooperation with a communications module (not shown), comprises suitable hardware to enable communication of information via PSTN (Public Switched Telephone Networks), POTS, Integrated Services Digital Network (ISDN), Ethernet, Fiber, DSL/ADSL, Wi-Fi, cellular (e.g., 3G, 4G, 5G, Global System for Mobile Communications (GSM), General Packet Radio Service (GPRS),etc.), Bluetooth, near field communications (NFC), Zigbee, among others, using TCP/IP, UDP, HTTP, DSL.

The user interface(s) 56 may include a keyboard, scroll-wheel, mouse, microphone, immersive head set, and/or display device(s) for enabling the presenting of user interfaces, including the rule engine configuration interface 10, among the other interfaces described above in association with FIGS. 1-3). The user interface(s) 56 enable input and/or output by or to a user, and/or visualization to a user. In some embodiments, the user interface(s) 56 may cooperate with associated software to enable augmented reality or virtual reality. The user interface(s) 56, when comprising a display device, enables the display of the various configuration screens described above. In some embodiments, the user interface(s) 56 may be coupled directly to the data bus 54.

The sources 58 include storage (e.g., non-transitory computer readable medium) for the sources described above, including the image/slide sources 44, biopsy procedure report 30, gross/macroscopy report 32, and LIS 34. In some embodiments, the sources 58 may be accessed over one or more networks via I/O interfaces 50.

The memory 52 may include any one or a combination of volatile memory elements (e.g., random-access memory RAM, such as DRAM, and SRAM, etc.) and nonvolatile memory elements (e.g., ROM, Flash, solid state, EPROM, EEPROM, hard drive, tape, CDROM, etc.). The memory 52 may store a native operating system, one or more native applications, emulation systems, or emulated applications for any of a variety of operating systems and/or emulated hardware platforms, emulated operating systems, etc. In some embodiments, a separate storage device (STOR DEV) may be coupled to the data bus 54 or as a network-connected device (or devices) via the I/O interfaces 50 and one or more networks. The storage device may be embodied as persistent memory (e.g., optical, magnetic, and/or semiconductor memory and associated drives).

In the embodiment depicted in FIG. 6, the memory 52 comprises an operating system 60 (OS) (e.g., LINUX, macOS, Windows, etc.), rule engine slide preprocessing and filtering process with AI integration software 24, a configuration GUI 62, and an AI engine library 64. The rule engine slide preprocessing and filtering process with AI integration software 24 includes a rule engine software 28, which includes a plurality of (software) modules for performing various functionality described above in association with FIGS. 3-5, including OCR software 36, HL7 software 37, slide metadata and image processing engine 38, and rule configuration and filtering engine 40. Functionality of the software modules 24-40 are described above in association with FIGS. 3-5, and hence description of the same here is omitted for brevity. The configuration GUI 62 renders one or more user interfaces for configuring the classification rules, as explained above in association with FIGS. 1-3 and omitted here for brevity. The AI engine library 64 includes plural machine learning methods/algorithms/models (e.g., Algorithm A, algorithm B, etc.) that may include third party algorithms. Algorithms may include FCNN, CNN, among other types of machine learning including foundation models, support vector machines, decision trees, random forests, K-nearest neighbor, etc. Note that the models described herein may also be referred to as networks (e.g., fully convolutional neural networks) or machine learning methods or algorithms. In some embodiments, all or a part of the functionality of the AI library 64 may be included in the rule configuration and filtering engine 40, or the rule configuration and filtering engine 40 cooperates with the AI library in some embodiments in fulfilling the AI algorithm functions.

Note that the memory 52 and storage may each be referred to herein as a non-transitory, computer readable (storage) medium or the like.

Execution of the software 24-40 and 62-64 may be implemented by the one or more processors 48 under the management and/or control of the operating system 60.

In one embodiment, the computing device 46 may be implemented using a dedicated or general purpose computer, including one or more co-located or network-connected processors. For instance, the computing device 46 may include one or more multi-core processors such as GPU (graphics processor unit) or TPU (tensor processing unit). In one embodiment, functionality of the computing device 46 may be implemented as one or more server(s) communicatively coupled in a communication network such as in a Cloud or server architecture. For instance, computing for the machine learning or artificial intelligence functionality may be via a local computing resource, or implemented remotely via one or more servers. In some embodiments, one or more of the functionality of the computing device 46 may be implemented using hardware components such as FPGAs or ASICs.

When certain embodiments of the computing device 46 are implemented at least in part with software (including firmware), it should be noted that the software can be stored on a variety of non-transitory computer-readable (storage) medium for use by, or in connection with, a variety of computer-related systems or methods. In the context of this document, a computer-readable medium may comprise an electronic, magnetic, optical, or other physical device or apparatus that may contain or store a computer program (e.g., executable code or instructions) for use by or in connection with a computer-related system or method. The software may be embedded in a variety of computer-readable mediums for use by, or in connection with, an instruction execution system, apparatus, or device, such as a computer-based system, processor-containing system, or other system that can fetch the instructions from the instruction execution system, apparatus, or device and execute the instructions.

When certain embodiments of the computing device 46 are implemented at least in part with hardware, such functionality may be implemented with any or a combination of the following technologies, which are all already existing in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), TPUs, GPUs, and/or other accelerators/co-processors, etc.

One having ordinary skill in the art should appreciate in the context of the present disclosure that the computing device 46 is merely illustrative of one embodiment, and that some embodiments of computing devices may comprise fewer or additional components, and/or some of the functionality associated with the various components depicted in FIG. 6 may be combined, or further distributed among additional modules or computing devices, in some embodiments. It should be appreciated that certain industry-recognized components of computer systems are omitted here to avoid obfuscating more relevant features of the computing device 46. Further, in some embodiments, the computing device 46, UI 56, and sources 58 or a subset thereof embody a system.

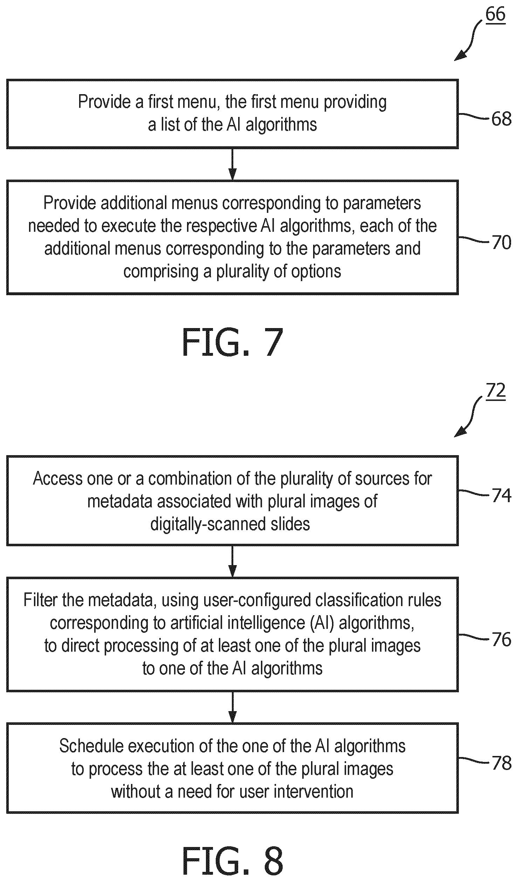

In view of the above description, it should be appreciated that one embodiment of an example method of providing a user interface for rule engine configuration for AI algorithms, denoted as method 66 (e.g., as executed by one or more processors 48, of the computing device 46, executing instructions) and shown in FIG. 7, includes providing a first menu, the first menu providing a list of the AI algorithms (68), and providing additional menus corresponding to parameters needed to execute the respective AI algorithms, each of the additional menus corresponding to the parameters and comprising a plurality of options (70).

In some embodiments, the additional menus may be auto-populated based on user selection of one of the AI algorithms from the list, the auto-population comprising a selection from among the plurality of options and subsequent listing of the selected options among the additional menus that are needed to execute the one of the AI algorithms selected from the list. In some embodiments, the GUI further includes an option to enable the user to create a classification rule beyond that which is available from the first menu, the additional menus, or a combination of the first menu and the additional menus. In some embodiments, the parameters include a supported application, a procedure type, a tissue type, and a stain type. In some embodiments, the GUI provides a list of the classification rules responsive to the configuration by the user.

In view of the above description, it should be appreciated that one embodiment of an example method for scheduling execution of AI algorithms, denoted as method 72 (e.g., as executed by one or more processors 48, of the computing device 46, executing instructions) and shown in FIG. 8 includes accessing one or a combination of the plurality of sources for metadata associated with plural images of digitally-scanned slides (74), filtering the metadata, using user-configured classification rules corresponding to artificial intelligence(AI) algorithms, to direct processing of at least one of the plural images to one of the AI algorithms (76), and scheduling execution of the one of the AI algorithms to process the at least one of the plural images without a need for user intervention (78).

In some embodiments, the method further includes determining that one of the plurality of sources is incapable of fully providing the metadata needed for execution of the one of the AI algorithms; and responsive to the determination, accessing the combination of the plurality of sources. In some embodiments, the method further includes accessing the combination of the plurality of sources according to a pre-configured hierarchy and availability of the metadata. In some embodiments, the plurality of sources includes any combination of a lab information system, a hospital information system, biopsy procedure reports, grossing reports, macroscopy reports, or storage having results of image content analysis as the metadata. In some embodiments, the method further includes filtering the metadata, using the user-configured classification rules corresponding to artificial intelligence(AI) algorithms, to direct processing of additional images among the plural images to respective AI algorithms selected from among the AI algorithms; and scheduling execution of the respective AI algorithms to process the additional images without a need for user intervention. In some embodiments, the method further includes, based on the filtering, limiting the scheduling to a subset of the plural images.

Certain embodiments of a rule engine configuration interface and associated rules-based filtering and scheduling methods are disclosed that may provide one or more of the following benefits. For instance, certain embodiments of a rule engine configuration interface and associated rules-based filtering and scheduling methods may increase the efficiency of the diagnostic process (thereby reducing the turnaround time on reporting), automate the process of filtering the relevant slide towards an algorithm, automate the process of obtaining the needed input for running the filtering rules, and/or routing relevant filtered slides towards the AI algorithm (e.g., in real time).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. Note that various combinations of the disclosed embodiments may be used, and hence reference to an embodiment or one embodiment is not meant to exclude features from that embodiment from use with features from other embodiments. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical medium or solid-state medium supplied together with or as part of other hardware, but may also be distributed in other forms. Any reference signs in the claims should be not construed as limiting the scope, and are used as an example illustration.

## Claims

1. A computing device (46) having access to a plurality of sources, the computing device comprising:
a memory (52) storing instructions (24);
a processor (48) configured to execute the instructions to cause the processor to:
access one or a combination of the plurality of sources for metadata associated with plural images of digitally-scanned slides (74);
filter the metadata, using user-configured classification rules corresponding to artificial intelligence(AI) algorithms, to direct processing of at least one of the plural images to one of the AI algorithms (76); and
schedule execution of the one of the AI algorithms to process the at least one of the plural images without a need for user intervention (78).

2. The computing device of claim 1, wherein the processor is further configured to execute the instructions to:
determine that one of the plurality of sources is incapable of fully providing the metadata needed for execution of the one of the AI algorithms; and
responsive to the determination, cause the processor to access the combination of the plurality of sources.

3. The computing device of any one of the preceding claims, wherein the processor is further configured to execute the instructions to:
access the combination of the plurality of sources according to a pre-configured hierarchy and availability of the metadata.

4. The computing device of any one of the preceding claims, wherein the plurality of sources includes any combination of a lab information system, a hospital information system, biopsy procedure reports, grossing reports, macroscopy reports, or storage having results of image content analysis as the metadata.

5. The computing device of any one of the preceding claims, wherein the processor is further configured to execute the instructions to:
filter the metadata, using the user-configured classification rules corresponding to artificial intelligence(AI) algorithms, to direct processing of additional images among the plural images to respective AI algorithms selected from among the AI algorithms; and
schedule execution of the respective AI algorithms to process the additional images without a need for user intervention

6. The computing device of any one of the preceding claims, wherein based on the filtering, the processor is further configured to execute the instructions to limit the scheduling to a subset of the plural images.

7. The computing device of any one of the preceding claims, wherein the processor is further configured to execute the instructions to provide a (GUI) arranged to enable a user to configure the classification rules, the GUI comprising:
a first menu providing a list of the AI algorithms; and
additional menus corresponding to parameters needed to execute the respective AI algorithms, each of the additional menus corresponding to the parameters and comprising a plurality of options.

8. The computing device of any one of the preceding claims, wherein the processor is further configured to execute the instructions to auto-populate the additional menus based on user selection of one of the AI algorithms from the list, the auto-population comprising a selection from among the plurality of options and subsequent listing of the selected options among the additional menus that are needed to execute the one of the AI algorithms selected from the list.

9. The computing device of any one of the preceding claims, wherein the GUI further includes an option to enable the user to create a classification rule beyond that which is available from the first menu, the additional menus, or a combination of the first menu and the additional menus.

10. The computing device of any one of the preceding claims, wherein the parameters include a supported application, a procedure type, a tissue type, and a stain type.

11. The computing device of any one of the preceding claims, wherein the GUI provides a list of the classification rules responsive to the configuration by the user.

12. A system comprising the plurality of sources and the computing device of any one of the preceding claims.

13. A method (66) for enabling a user to configure the classification rules of any one of the preceding claims.

14. A method (72) for scheduling execution of the one of the AI algorithms of any one of the preceding claims.

15. A non-transitory, computer readable medium (52) comprising the instructions, that when executed, causes the processor to implement the method of any one of the preceding claims.
